# EUROPEAN PATENT SPECIFICATION

(11) **EP 1 773 755 B1**
(45) Date of publication and mention of the grant of the patent: **31.10.2012**
(21) Application number: 04757298.7
(22) Date of filing: 28.07.2004
(51) Int. Cl.: C07C 233/00, C08J 9/00, C08G 18/08

(54) **PROCESS FOR THE PRODUCTION OF POLYISOCYANATES**
VERFAHREN ZUR HERSTELLUNG VON POLYISOCYANATEN
METHODE DE PRODUCTION DE POLYISOCYANATES

(43) Date of publication of application: 18.04.2007
(60) Divisional application: 12164242.5
(73) Proprietor: HUNTSMAN INTERNATIONAL LLC, Salt Lake City, Utah 84108 (US)
(72) Inventor: SMITH, Richard Colin, Gwynned LL57 4UP (GB); CARR, Robert, Henry, 3060 Bertem (BE)
(74) Representative: Swinnen, Anne-Marie
(86) International application number: PCT/US2004/024071
(87) International publication number: WO 2006/022641

(56) References cited:
- US-A- 4 324 879
- US-A- 4 405 527
- US-A- 5 990 184
- US-A- 6 063 826

## Description

This invention relates to a process for the preparation of organic polyisocyanates in the presence of solvents in which the solvent is reused.

Organic polyisocyanates are produced on a large industrial scale by phosgenation of the corresponding primary polyamines in the presence of inert organic solvents such as chlorobenzene or orthodichlorobenzene.

In the preparation of the industrially important polyisocyanates, particularly in the preparation of hexamethylene diisocyanates, tolylene diisocyanates or polyisocyanates of the diphenyl methane series by phosgenation of the corresponding di- and polyamines, traces of byproducts containing isocyanate groups are invariably formed (e.g. 6-chloro-hexylisocyanate in the preparation of hexamethylene diisocyanate, tolyl isocyanate in the preparation of tolylene diisocyanates and phenyl isocyanate in the preparation of polyisocyanates of the diphenyl methane series by the phosgenation of aniline/formaldehyde condensates). Such unwanted isocyanate compounds seriously impair the quality of the desired end products (polyisocyanates). It has therefore been attempted to remove these impurities from the polyisocyanate by distillation together with the solvent after the phosgenation reaction and subsequently free the solvent from these impurities by an elaborate column distillation. The solvent can then be reused. This purification of the solvent by distillation requires considerable consumption of energy and expenditure in apparatus, and particular difficulties are encountered when the compounds have boiling points close to those of the solvents used.

US 4405527 describes a process for the preparation of polyisocyanates in the presence of solvents, in which the solvent is freed from traces of compounds containing isocyanate groups before it is reused. The solvent is treated with compounds containing isocyanate reactive hydrogen atoms, such as alcohols or amines, to convert the readily volatile isocyanates into reaction products containing urethane or urea groups. The treated solvent is then separated from these reaction products by distillation. Even though these reaction products (which have much higher boiling points than the trace isocyanates) are much more easily separated by distillation, removal of these by-products necessitates distillation of the whole quantity of solvent required for the preparation of the polyamine solution. This entails a high expenditure of energy due to the large quantity of solvent required.

If the ureas or urethanes formed in the process described in US 4405527 are not removed by distillation, they enter the phosgenation process when the solvent is subsequently reused and will readily undergo numerous further reactions with phosgene and with the newly formed isocyanates. The total polyisocyanate yield, the yield of separated diisocyanate product if produced and the quality of the polyisocyanates are thereby diminished. Also the urethanes or ureas formed could, during subsequent polyurethane formation using the end product polyisocyanates, regenerate phenylisocyanate by thermal cleavage.

In US 4745216 the solvent to be freed from traces of isocyanate and to be reused is treated with certain polymers and then separated mechanically (e.g. by decanting or filtration) from these polymers. The polymers employed are crosslinked polymers which are insoluble in the solvent and contain at least one functional group selected from primary alcoholic hydroxyl groups, secondary alcoholic hydroxyl groups, primary amino groups and secondary amino groups. Here also a waste-stream is generated.

It is therefore an object of the present invention to provide a new process for the removal of traces of isocyanate from the solvent leaving the process for the production of polyisocyanates, in which a solvent free from traces of isocyanate and/or with reduced impurity level could be obtained without elaborate distillation of the solvent, recovered and reused in conventional industrial processes for the preparation of polyisocyanates.

It was surprisingly found that this problem of isocyanates in the solvent to be reused could be solved by treating the solvent which was to be freed from traces of isocyanate with isocyanate trimerisation catalysts.

The low molecular weight mono-isocyanates are thereby converted into the thermally stable, high molecular weight trimers. Mixed trimers can also be formed derived from reacting different isocyanate-group-containing by-products.
In the preparation of polyisocyanates of the diphenyl methane series the presence of the trimers does not have any deleterious effects on the quality of the polyisocyanates produced by the process and hence they do not have to be removed from the solvent before re-using it. It is preferred in other embodiments (e.g. in the preparation of tolylene diisocyanates) to remove the trimer stream from the solvent before re-using it; such a trimer waste stream is however safer to handle than a waste stream of mono-isocyanates which are much more volatile than the trimers.

The process of the present invention has the further advantage that the polyamines used as starting materials in the phosgenation reaction may contain a higher proportion of by-products, which would result in formation of a correspondingly higher proportion of isocyanate group-containing compounds to be removed than could be tolerated in known processes. As a result, the cost in energy and outlay in apparatus required for the preliminary preparation of the polyamine starting material is considerably reduced in the process of the present invention.

The process of the present invention can be applied in the production of any type of organic polyisocyanate. Particular preference goes to the aromatic polyisocyanates such as diphenylmethane diisocyanate in the form of its 2,4'-, 2,2'- and 4,4'-isomers and mixtures thereof, the mixtures of diphenylmethane diisocyanates (MDI) and oligomers thereof known in the art as "crude" or polymeric MDI (polymethylene polyphenylene polyisocyanates) having an isocyanate functionality of greater than 2, toluene diisocyanate in the form of its 2,4- and 2,6-isomers and mixtures thereof, 1,5-naphthalene diisocyanate and 1,4-diisocyanatobenzene. Other suitable organic polyisocyanates, which may be mentioned, include the aliphatic diisocyanates such as isophorone diisocyanate, 1,6-diisocyanatohexane and 4,4'-diisocyanatodicyclohexylinethane.

Most preferably the present process is applied in the production of polyisocyanates of the diphenyl methane series.
In such a case the low molecular weight impurities containing isocyanate groups are primarily, but not exclusively, phenylisocyanate, cyclohexyl isocyanate and o- and p-chloromethyl phenylisocyanate.

The present invention relates to a process for the preparation of polyisocyanates by the reaction of polyamines from which the polyisocyanates are derived preferably as solutions in an inert solvent with phosgene optionally as a solution in an inert solvent by single stage or multi-stage phosgenation reaction or any variation known in the art, in batch, continuous or semi-continuous modes, at atmospheric pressure or above. After completion of the phosgenation reaction, the reaction mixture is distilled. The solvent is then treated to remove traces of isocyanate and reused for the preparation of amine solution and/or phosgene solution.
In this process, the whole quantity of solvent recovered may be treated but it is also possible to treat only part of the solvent used for the preparation of amine solution by this method.

Particular embodiments of the present invention include:
(i) stepwise distillation of the phosgenation reaction mixture to prepare a solvent stream particularly enriched in isocyanate impurities which is subsequently treated to remove traces of isocyanate;
(ii) further partial treatment of the solvent removed from the phosgenation reaction mixture, either by further distillation or any other known method, to prepare a solvent stream particularly enriched in isocyanate impurities which is subsequently treated to remove traces of isocyanate;
(iii) return of the solvent which has been treated to remove isocyanate impurities to another suitable part of the isocyanate production plant, for example, a phosgenation reactor or the solvent distillation vessel;
(iv) incomplete trimerisation of the isocyanate impurities in the solvent stream in as much as ultra low levels of the impurities can be tolerated in the recycled solvent;
(v) inclusion of di-isocyanate to a pre-defined level, such as but not limited to mixtures of the 4,4'-, 2,4'- and 2,2'-MDI isomers, within the mixture to be trimerised to produce compounds which have trimer structures composed of mixtures of isocyanate impurities and di-isocyanate moieties and, thus, untrimerised isocyanate groups capable of further reaction for example, when forming a polyurethane material from the polyisocyanate product which contains the trimerised material;
(vi) operation of any or all of the above described processes or sub-units of operation in either batch, continuous or semi-continuous modes, at atmospheric pressure or above.

The principle employed in the process of the present invention for working up the solvent is particularly suitable for a multi-stage process for the preparation of polyisocyanates, composed of the following individual stages:
(a) reaction of (i) solutions of the polyamine(s) underlying the polyisocyanate(s) in an inert solvent with (ii) phosgene optionally as a solution in an inert solvent in a single stage or multi-stage reaction of phosgenation;
(b) separation of the excess phosgene and of the hydrogen chloride formed from the liquid reaction mixture obtained by (a);
(c) separation of the solvent together with readily volatile compounds containing isocyanate groups from the solution obtained in (b) by evaporation and recovery of the product of the process as evaporation residue which is optionally subjected to a further process of distillation;
(d) recovery of a solvent containing volatile isocyanate compound(s) by condensation of the vapors obtained in (c) and reuse of part of the condensate for the preparation of amine solution (i) and optionally of another part of the condensate for the preparation of phosgene solution (ii).

The phosgenation reaction is carried out in any known manner, using solutions of polyamines in inert solvents and phosgene optionally as solution in inert solvents. In the process of the present invention, this phosgenation reaction may be carried out either in one stage or in several stages. For example, phosgenation may be carried out by forming suspensions of carbamic acid chlorides at low temperatures and then converting these suspensions into polyisocyanate solutions at elevated temperatures ("cold/hot phosgenation"). Particularly suitable polyamine starting materials are the technically important polyamines such as hexamethylene diamine; 2,4- and/or 2,6-diamino toluene; 2,4'-, 2,2'- and 4,4'-diaminodiphenyl methane and their mixtures with higher homologues (known as "polyamine mixtures of the diphenyl methane series") which may be obtained in known manner by aniline/formaldehyde condensation; 1,5-diaminonaphthalene; 1-amino-3,3,5-trimethyl-5-aminomethyl-cyclohexane (isophorone diamine); tris-(isocyanatophenyl)-methane and perhydrogenated diaminodiphenyl methanes and their mixtures with higher homologues.

In the process of the present invention, the amine starting materials such as those mentioned as examples above may be used in the form of 3 to 50 wt%, preferably 5 to 40 wt% solutions in inert solvents. The phosgene required for the phosgenation reaction is generally used in the form of a 10 to 60 wt%, preferably 25 to 50 wt% solution in inert solvents or, optionally, without solvent.

Suitable inert solvents both for the polyamine and for phosgene are known to those in the art. Exemplary solvents are chlorinated aryl and alkylaryl hydrocarbons such as monochlorobenzene (MCB), o-dichlorobenzene, trichlorobenzene and the corresponding toluene, xylene, methylbenzene and naphthalene compounds, and many others known in the art such as toluene, xylenes, nitrobenzene, ketones, and esters. Specific examples of appropriate solvents are mono- and dichlorobenzene.

After the phosgenation has been carried out by methods known in the art, the excess phosgene and the hydrogen chloride formed are removed by methods known in the art, such as by blowing them out with inert gas or by partial distillation. The phosgenation product present in the form of a solution is then separated, either simply by evaporation or by fractional distillation, into a gaseous phase containing solvent together with volatile compounds with isocyanate groups and a liquid phase substantially made up of crude polyisocyanate. The liquid phase obtained may, if desired, be worked up by distillation in known manner if a pure polyisocyanate is to be produced. This separation of crude polyisocyanate and volatile compounds is generally carried out at a temperature of from 80 to 220°C (preferably from 120 to 190°C) at a pressure of from 10 to 4000 mbar (preferably from 100 to 3000 mbar).

The vapor containing solvent together with volatile compounds with isocyanate groups is condensed to form a solvent condensate containing volatile isocyanates, in particular monoisocyanates. The quantity of isocyanate compounds present in the condensate (calculated as NCO with molecular weight 42) may amount to 50 to 5000 ppm, in particular 100 to 1500 ppm (by weight).

The obtained solvent condensate is then treated with isocyanate trimerisation catalyst in order to convert any traces of isocyanate by-products.

Any compound that catalyses the isocyanate trimerisation reaction can be used as trimerisation catalyst such as tertiary amines, triazines and metal salt trimerisation catalysts. Examples of suitable metal salt trimerisation catalysts are alkali metal salts of organic carboxylic acids. Preferred alkali metals are potassium and sodium. And preferred carboxylic acids are acetic acid and 2-ethylhexanoic acid.
Particularly preferred trimerisation catalysts are 1,3,5-tris (3-(dimethylamino)propyl) hexahydro-s-triazine (commercially available as Polycat 41 from Air Products) or tris (dimethylaminomethyl) phenol (commercially available as DABCO TMR-30 from Air Products).
Another preferred group of catalysts are so-called "reactive amines" containing additional isocyanate-reactive groups (OH, NH or NH₂) for reaction with isocyanate. Suitable examples include N,N-dimethylaminoethyl-N'-methyl ethanolamine (commercially available as DABCO T from Air Products). Such catalysts may be used as such for trimerisation or, optionally, pre-reacted with any isocyanate-containing compound.
One or more solvents for the catalyst may also be employed.

Treatment of the condensate with the above-described trimerisation catalysts is generally carried out within the temperature range of from 10°C to 150 °C, preferably from 25°C to 150°C, more preferably from 30°C to 60°C.
The preferred tertiary amine catalysts can be inactivated by salt formation with, for example, chloride from residual hydrogen chloride or other trace impurities with labile chlorine atoms. Therefore, to achieve close to quantitative removal of the compounds containing isocyanate groups, the trimerisation catalysts should be used in at least molar excess of the total inactivating species which can easily be determined experimentally by the person skilled in the art. The progress of the trimerisation can be followed by monitoring the exotherm generated. The excess trimerisation catalyst can be inactivated either by addition of additional hydrogen chloride or by reaction with hydrogen chloride present in the phosgenation reaction mixture.

In one embodiment, the solvent is then reused for preparing a fresh batch of amine and/or phosgene solutions. In contrast to the known methods used in the art, however, subsequent purifications by distillation for removing compounds containing isocyanate groups and/or separation of the obtained reaction products of these by-products are not necessary in the process of the present invention.
However one can of course remove the solvent from the obtained trimer before re-using it. This is then preferably done by fractional distillation.

When the solvent has been treated, it may be used again for the preparation of amine solution (i) and/or phosgene solution (ii). As a result, when the solvent containing volatile isocyanate compounds is treated in accordance with the process of this invention polyisocyanates with a sharply reduced content of readily volatile isocyanate components are obtained.

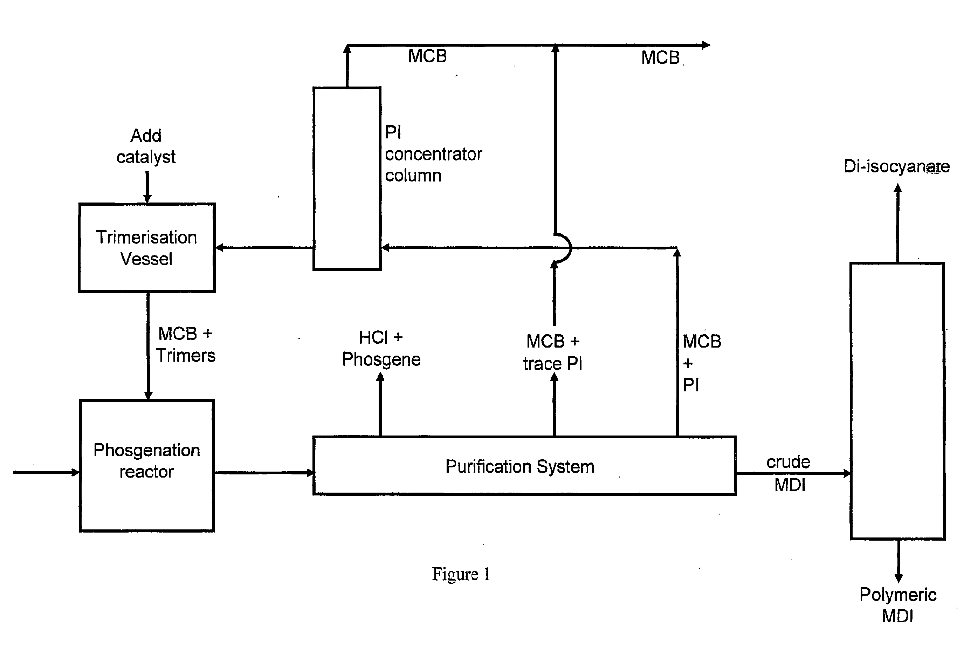

A particularly preferred embodiment of the present invention involving preparation of MDI will be described here in connection with the block diagram provided as Figure 1.
The reaction mixture obtained at the end of the phosgenation train is first purified in 3 stages. First excess phosgene and hydrogen chloride are removed. Then MCB solvent containing traces of phenylisocyanate (PI) (100 to 200 ppm) is removed; solvent recovered in this step goes back in the reaction train untreated. In a last purification MCB solvent enriched in PI and other trace isocyanate impurities is separated from crude MDI; the crude MDI is subsequently distilled into a polymeric fraction and a difunctional fraction.
The enriched MCB fraction is continuously fed into the PI concentrator column. When the PI concentration in the bottom of this column has reached ca. 10 -30% PI in MCB (preferably 15- 25%) as determined by the atmospheric boiling point of the mixture, typically from 140 to 160°C (preferably 140 to 155°C), the liquid is transferred to the trimerisation vessel, either continuously or, preferably, as a batch. One or more batches of the concentrated liquid can be transferred as required. After cooling the liquid, Polycat 41 is added in slight molar excess over the deactivating species (typically 4 litres of Polycat 41 per 800 litres of liquid). The temperature before catalyst addition is typically 10 to 100°C (preferably 20 to 40°C). The process of trimerisation after catalyst addition can be followed by monitoring the exotherm which increases the temperature to 35 to 70°C, typically 50°C. After 2 to 12 hours, the liquid consisting of MCB, various trimers and some residual unreacted isocyanates is transferred back to the reaction system, optionally over a short time period (e.g. less than one hour) but preferably over the course of many hours (typically 6 - 12 hours).

By the present process polyisocyanates are obtained having very low levels of isocyanate-containing impurities.
For example, in the preparation of polyisocyanates of the diphenyl methane series, MDI isomers and polymeric MDI containing very low levels of phenylisocyanate are obtained, generally below 50 ppm or even below 10 ppm.

## Claims

1. A process for the production of a polyisocyanate comprising (a) phosgenating a polyamine to form a polyisocyanate by reacting (1) a polyamine on which the polyisocyanate is based in solution in an inert solvent with (2) phosgene optionally in solution in an inert solvent, (b) separating the solvent from the polyisocyanate, (c) treating the separated solvent with an isocyanate trimerisation catalyst.

2. Process according to claim 1 wherein the polyisocyanate is an aromatic polyisocyanate.

3. Process according to claim 2 wherein the polyisocyanate is of the diphenyl methane series.

4. Process according to any one of claims 1 to 3 wherein monochlorobenzene or o-dichlorobenzene is the solvent treated in (c).

5. Process according to any one of the preceding claims wherein the isocyanate trimerisation catalyst is 1,3,5-tris (3-(dimethylamino)propyl) hexahydro-s-triazine or tris (dimethylaminomethyl) phenol.

6. Process according to any one of the preceding claims wherein step (c) is carried out at a temperature of from 10°C to 150°C.

## Patentansprüche

1. Verfahren zum Herstellen eines Polyisocyanats,
das Folgendes aufweist:
(a) Phosgenieren eines Polyamins, um ein Polyisocyanat zu erzeugen, indem (1) ein Polyamin, auf dem das Polyisocyanat basiert, in Lösung in einem inerten Lösungsmittel mit (2) Phosgen, gegebenenfalls in Lösung in einem inerten Lösungsmittel, umgesetzt wird,
(b) Abtrennen des Lösungsmittels vom Polyisocyanat,
(c) Behandeln des abgetrennten Lösungsmittels mit einem Isocyanat-Trimerisierungskatalysator.

2. Verfahren nach Anspruch 1,
wobei das Polyisocyanat ein aromatisches Polyisocyanat ist.

3. Verfahren nach Anspruch 2,
wobei das Polyisocyanat aus der Diphenylmethan-Reihe ist.

4. Verfahren nach einem der Ansprüche 1 bis 3,
wobei das unter (c) behandelte Lösungsmittel Monochlorbenzol oder o-Dichlorbenzol ist.

5. Verfahren nach einem der vorstehenden Ansprüche,
wobei der Isocyanat-Trimerisierungskatalysator 1,3,5-Tris-(3-(dimethylamino)propyl)hexahydro-s-triazin oder Tris(dimethylaminomethyl)phenol ist.

6. Verfahren nach einem der vorstehenden Ansprüche,
wobei der Schritt (c) bei einer Temperatur von 10 bis 150 °C durchgeführt wird.

## Revendications

1. Procédé pour la production d'un polyisocyanate, comprenant (a) la phosgénation d'une polyamine pour former un polyisocyanate en faisant réagir (1) une polyamine servant de base au polyisocyanate en solution dans un solvant inerte avec (2) du phosgène facultativement en solution dans un solvant inerte, (b) la séparation du solvant du polyisocyanate, (c) le traitement du solvant séparé avec un catalyseur de trimérisation d'isocyanate.

2. Procédé suivant la revendication 1, dans lequel le polyisocyanate est un polyisocyanate aromatique.

3. Procédé suivant la revendication 2, dans lequel le polyisocyanate est de la série du diphénylméthane.

4. Procédé suivant l'une quelconque des revendications 1 à 3, dans lequel le monochlorobenzène ou le o-dichlorobenzène est le solvant traité en (c).

5. Procédé suivant l'une quelconque des revendications précédentes, dans lequel le catalyseur de trimérisation d'isocyanate est la 1,3,5-tris(3-diméthylamino)-propyl)hexahydro-s-triazine ou le tris(diméthylaminométhyl)phénol.

6. Procédé suivant l'une quelconque des revendications précédentes, dans lequel l'étape (c) est mise en ouvre à une température de 10°C à 150°C.
